# EUROPEAN PATENT APPLICATION

(11) **EP 3 922 239 A2**
(43) Date of publication of application: **15.12.2021**
(21) Application number: 21168717.3
(22) Date of filing: 15.04.2021
(51) Int. Cl.: A61K 9/06, A61K 9/00, A61K 31/164, A61K 31/728, A61K 38/20, A61K 47/32, A61P 29/00, A61Q 19/08, A61K 8/04, A61K 8/42, A61K 8/64, A61K 8/73

(54) **A GEL COMPOSITION FOR TOPICAL APPLICATION FOR THE REDUCTION OF LOCAL INFLAMMATION**

(30) Priority: 16.04.2020 CH 4572020
(71) Applicant: Contrad Swiss SA, 6900 Lugano (CH)
(72) Inventor: SERRENTINO, Joanne, SAINTE-CATHERINE-DE-HATELY QC JOB1WO (CA)
(74) Representative: Comoglio, Elena

(57) **Abstract**

The invention relates to a gel composition for topical application suitable for reducing local inflammation, the composition being characterised by a combination of a biomimetic peptide, hyaluronic acid, panthenol, a gelling agent, and water, as well as, optionally, one or more further optional ingredients selected from the group consisting of thickening agents, wetting agents, solubilizing agents, chelating agents, pH adjusting agents, preservative agents, and any combination thereof.

## Description

The present invention generally relates to the therapeutic sector and more specifically to a gel composition for topical application for reducing local inflammation, having a strong soothing action effective against stiffness and states of muscle tension that cause inflammation and pain.

The composition of the present invention is characterized, i.a., in that it contains a specific biomimetic oligopeptide shown to be capable of exerting an extremely beneficial action on local inflammation states, particularly when combined with the additional active ingredients present in the composition.

Oligopeptides generally have a molecular weight of less than 5,000 Daltons and consist of an amino acid chain which often "mimics" active parts of proteins normally found in the human body. This is why they are called "biomimetic" oligopeptides, as they are able to bind to specific cellular receptors and consequently regulate various biological processes.

The advantage of using the oligopeptides, in addition to their proven functionality, is their total safety of use, since these oligopeptides faithfully mimic parts of molecules normally found in the human body. The possibilities for the development of biomimetic oligopeptides are countless, as well as the possibilities for the creation of a mix of oligopeptides with specific and, in any case, complementary actions, or for the selection of specific oligopeptides according to the specific activity required.

The present invention provides a gel composition for topical application as defined in the attached claim 1, which has a combination of active ingredients particularly effective in reducing local inflammation.

The dependent claims define further features and advantages of the composition according to the present invention.

The claims form an integral part of the present specification.

Thanks to its particular combination of ingredients, the composition of the present invention was shown to be particularly effective in facilitating the movement of joints and tendons for greater mobility and flexibility.

The composition according to the present invention comprises the combination of the following components:
- a peptide component, consisting of a biomimetic oligopeptide designated as SH-Polypeptide 6;
- high molecular weight (HMW) hyaluronic acid;
- panthenol;
- a gelling agent; and
- water.

SH-Polypeptide 6 is a single-chain recombinant human peptide having a maximum length of 160 amino acids and comprising the amino acid sequence SEQ ID NO:1. It may include disulfide bridges and/or glycosylation. SH-Polypeptide 6 is manufactured through a recombinant technique known per se, including, for example, fermentation by a prokaryotic expression system, such as *E. coli,* by using a starting gene which is the synthetic copy of the human gene encoding for Interleukin10, used as such or adapted to the expression system used as the host.

SEQ ID NO:1: SPGQGTQSEN SCTHFPGNLP NMLRDLRDAF SRVKTFFQMK
DQLDNLLLKE SLLEDFKGYL GCQALSEMIQ FYLEEVMPQA ENQDPDIKAH
VNSLGENLKT LRLRLRRCHR FLPCENKSKA VEQVKNAFNK LQEKGIYKAM
SEFDIFINYI EAYMTMKIRN

According to a preferred embodiment, SH-Polypeptide 6 consists of the amino acid sequence of SEQ ID No: 1.

IL-10 is a multifunctional cytokine with a strong anti-inflammatory effect, which exerts a plethora of immunomodulatory functions during an inflammatory response and is particularly important during the resolution phase [1].

The composition of the present invention also contains high molecular weight hyaluronic acid (preferably higher than 1000 kDaltons, for example between 1000 and 5000 kDaltons), preferably in the form of a salt such as, for example, sodium hyaluronate. This substance has remarkable anti-inflammatory properties [2] through regulation of pro-inflammatory genes [3] and action on inflammatory cells [4].

The composition of the present invention also contains panthenol, a precursor of Vitamin B5 which contributes to a soothing and calming action on the skin [5]. Panthenol has also been studied for its effect against inflammation [5], [6] and can be considered as a valid support for the anti-inflammatory activity.

The gelling agent used in the cosmetic composition of the present invention is a rheology modifier capable of changing a mixture from the liquid to the gel phase. Gelling agents particularly suitable for use in the cosmetic composition of the present invention, which is water-based, are acrylic polymers and derivatives thereof, such as for example polyacrylic acid polymers known under the trade name of Carbopol.

According to preferred embodiments, the cosmetic composition of the present invention also contains one or more of the following additional components: thickening agents, wetting agents, solubilizing agents, chelating agents, pH adjusting agents, and preservative agents.

An exemplary thickening agent suitable for use in the cosmetic composition of the invention is xanthan gum, which is a high molecular weight polysaccharide with a thickening and stabilizing action on the "gel" form.

Exemplary wetting agents suitable for use in the cosmetic composition of the invention are glycerin and propylene glycol; the latter is a so-called "skin enhancer" or "skin carrier", as it enhances the absorption of the product, and therefore also of its main functional ingredients into the skin. This ingredient can be replaced in the formulation in question by propanediol, which is plant-derived and therefore considered more skin friendly.

An exemplary solubilizing agent suitable for use in the cosmetic composition of the invention is PEG-40 hydrogenated castor oil. This ingredient can be used to solubilize as much as possible the ingredients of the formula, thus avoiding any risk of separation or chemical-physical instability.

Exemplary preservatives suitable for use in the cosmetic composition of the invention are dehydroacetic acid, benzoic acid, ethylhexylglycerin and phenoxyethanol; particularly if used in a mixture (a mixture called "EUXYL K701"), they can prevent any type of bacterial and/or fungal contamination of the formulation.

An exemplary chelating agent suitable for use in the cosmetic composition of the invention is ethylenediaminetetraacetic acid (EDTA) disodium salt, which is suitable to assist the preservative system by acting as a heavy metal chelator.

An exemplary pH adjusting agent suitable for use in the cosmetic composition of the invention is sodium hydroxide, which is introduced in quantities such as to adjust the pH of the formulation within the identified preferred range (pH between 5.00 and 5.70).

The preferred embodiments described above can be combined with each other as required, and the implementation of such combinations falls within the skills of the person skilled in the art.

The table below illustrates, by way of non-limiting example, suitable percentages (% w/w) of the aforementioned ingredients of a cosmetic composition falling within the scope of the present invention:

| **Ingredient** | **% w/w** |
|---|---|
| DEMINERALIZED WATER | > 75 |
| 99.8% GLYCERIN PF | 1-5 |
| HMW HYALURONIC ACID | 0.1-1 |
| SH-Polypeptide 6 | 0.0000001 - 0,1 |
| D-PANTHENOL USP | 0.1-1 |
| XANTHAN GUM | 0.1-1 |
| DISODIUM EDTA | 0.1-1 |
| CARBOPOL ULTREZ 30 | 1-5 |
| 30% SODIUM HYDRATE SOLUTION | <0.1 |
| PEG 40 HYDROGENATED CASTOR OIL | 0.1-1 |
| EUXYL K701 | 0.1-1 |
| MONOPROPYLENE GLYCOL | 0.1-1 |

The product does not contain perfuming substances, therefore it does not have any perfume allergens.

### Experimental Section

A formulation devised with the ingredients described in Table 1 and falling within the quantitative ranges described therein (hereafter designated as "500AI") was subjected to an anti-inflammatory efficacy case study on a 44-year-old woman suffering from inflammation and pain to the rotator cuff of the shoulder. The subject had suffered from pain to the shoulder and the whole arm for one month after shoulder surgery prior to treatment with 500AI. To assess the effectiveness of 500AI, several measurements of the inflammatory status, pain and mobility were carried out before and after treatment with AI500.

### 1) DASH (Disabilities of the Arm, Shoulder and Hand Questionnaire)

The DASH questionnaire is a self-administered region-specific outcome instrument developed as a measure of self-rated upper limb disability and symptoms. DASH mainly consists of a 30-item disability/symptom scale, scored 0 (no disability) to 100 [7]. The DASH questionnaire was completed before the application of 500AI, after 2 hours, and after 12 hours, to quantify the patient's state of disability and movement.

### 2) SF-MPQ (Short-Form McGill Pain Questionnaire)

To assess pain, the SF-MPQ was used, i.e., a questionnaire consisting of 15 descriptors (11 sensory; 4 affective) which are rated on an intensity scale as 0 = none, 1 = mild, 2 = moderate or 3 = severe [8]. The SF-MPQ questionnaire was completed before the application of 500AI, after 2 hours, and after 12 hours, to quantify the patient's pain status.

### 3) VAS (Visual Analogue Scale)

The pain VAS is a continuous scale comprised of a horizontal (HVAS) or vertical (VVAS) line, usually 10 centimetres (100 mm) in length, anchored by 2 verbal descriptors, one for each symptom extreme [9], [10]. VAS was used to assess pain in three situations: Activities of Daily Living (VAS/ADL), Sedentary Activities (VAS/SA), and at work (VAS/work). The three VAS questionnaires were completed before the application of 500AI, after 2 hours, and after 12 hours, to quantify the patient's pain status.

### 4) Infrared imaging

To assess shoulder inflammation, thermography was used, i.e., a technique that was shown to be useful for detecting inflammation characterized by changes in skin temperature [11], [12]. Measurements were carried out using an infrared camera (ICI DIGITAL THERMAL version TEi P V6. 8) in an environment where the temperature was 20°C and the humidity was 50% at a distance of 1 metre from the subject and with 0.96 emissivity. The ICI IR flash pro reporter, 75% grey option, Medical software was used. The shoulder image was recorded before applying 500AI and after 6 minutes.

The test results indicate that the composition of the present invention caused a decrease in pain and in the state of disability in the test area 2 hours after applying the product, with further decrease in pain after 12 hours from the application. Additionally, a decrease in shoulder surface temperature was observed using an infrared camera.
In particular, the following was observed:
- A reduction in the disability status of the shoulder, highlighted by the decrease in DASH scores (from a value of 89 before applying the product to a value of 56 after 2 hours). A further decrease in the DASH score to a value of 9.1 was detected after 12 hours (Table 1).
- A substantial reduction in pain 2 hours after application is clear from the SF-MPQ, VAS/ADL, VAS/SA and VAS/work scores (Table 1). The pain was reduced from an average value of 8.75 before application of the product to an average value of 5.2 after 2 hours and up to an average value of 2.2 after 12 hours (the SF-MPQ values were scaled by a factor of 4.5 before the average values were calculated) (Table 1). All four questionnaires showed similar reductions in pain scores, supporting the results obtained.
- A reduction in the skin temperature of the shoulder after 6 minutes of administering the product to the same area. Infrared measurements show that the initial skin temperature decreased after 6 minutes. The initial temperature was reduced in a first zone from 33.3°C at time zero to 31.2°C after 6 minutes, and in a second zone from 31.0°C at time zero to 30.3°C after 6 minutes.

**Table 1. Results of the three questionnaires to quantify the patient's pain and state of disability and movement.**

| | DASH (0-100) | SF-MPQ (0-45) | VAS/ADL (0-10) | VAS/SA (0-10) | VAS/work (0-10) |
|---|---|---|---|---|---|
| T0 | 89 | 44 | 8.1 | 7.9 | 9.2 |
| 2 hours | 56 | 28 | 5.2 | 5.1 | N/A |
| 12 hours | 9.1 | 4 | 2.9 | 1.8 | 3.1 |

### References

[1] J. M. Garcia et al., 'Role of Interleukin-10 in Acute Brain Injuries', Front. Neurol., vol. 8, 2017, doi: 10.3389/fneur.2017.00244.
[2] G. D. Albano et al., 'Effect of High, Medium, and Low Molecular Weight Hyaluronan on Inflammation and Oxidative Stress in an In Vitro Model of Human Nasal Epithelial Cells', Mediators of Inflammation, 2016. https://www.hindawi.com/journals/mi/2016/8727289/ (accessed Nov. 18, 2019).
[3] D. Jiang et al., 'Regulation of lung injury and repair by Toll-like receptors and hyaluronan', Nat. Med., vol. 11, no. 11, pp. 1173-1179, Nov. 2005, doi: 10.1038/nm1315.
[4] P. L. Bollyky et al., 'ECM components guide IL-10 producing regulatory T-cell (TR1) induction from effector memory T-cell precursors', Proc. Natl. Acad. Sci. U.S.A., vol. 108, no. 19, pp. 7938-7943, May 2011, doi: 10.1073/pnas.1017360108.
[5] F. Ebner, A. Heller, F. Rippke, and I. Tausch, 'Topical use of dexpanthenol in skin disorders', Am J Clin Dermatol, vol. 3, no. 6, pp. 427-433, 2002, doi: 10.2165/00128071-200203060-00005.
[6] W. Li-Mei, T. Jie, W. Shan-He, M. Dong-Mei, and Y. Peng-Jiu, 'Anti-inflammatory and Anti-oxidative Effects of Dexpanthenol on Lipopolysaccharide Induced Acute Lung Injury in Mice', Inflammation, vol. 39, no. 5, pp. 1757-1763, Oct. 2016, doi: 10.1007 /s10753-016-0410-7.
[7] C. Gummesson, I. Atroshi, and C. Ekdahl, 'The disabilities of the arm, shoulder and hand (DASH) outcome questionnaire: longitudinal construct validity and measuring self-rated health change after surgery', BMC Musculoskelet Disord, vol. 4, p. 11, Jun. 2003, doi: 10.1186/1471-2474-4-11.
[8] R. Melzack, 'The short-form McGill Pain Questionnaire', Pain, vol. 30, no. 2, pp. 191-197, Aug. 1987, doi: 10.1016/0304-3959(87)91074-8.
[9] G. A. Hawker, S. Mian, T. Kendzerska, and M. French, 'Measures of adult pain: Visual Analog Scale for Pain (VAS Pain), Numeric Rating Scale for Pain (NRS Pain), McGill Pain Questionnaire (MPQ), Short-Form McGill Pain Questionnaire (SF-MPQ), Chronic Pain Grade Scale (CPGS), Short Form-36 Bodily Pain Scale (SF-36 BPS), and Measure of Intermittent and Constant Osteoarthritis Pain (ICOAP)', Arthritis Care & Research, vol. 63, no. S11, pp. S240-S252, 2011, doi: 10.1002/acr.20543.
[10] E. C. Huskisson, 'Measurement of pain', Lancet, vol. 2, no. 7889, pp. 1127-1131, Nov. 1974, doi: 10.1016/s0140-6736(74)90884-8.
[11] I. Rossignoli, I. Femández-Cuevas, P. J. Benito, and A. J. Herrero, 'Relationship between shoulder pain and skin temperature measured by infrared thermography in a wheelchair propulsion test', Infrared Physics & Technology, vol. 76, pp. 251-258, May 2016, doi: 10.1016/j.infrared.2016.02.007.
[12] C. B. Sanchez, M. Martinez M, V. Campo C, and B. Cervero AV, 'Thermo-graphic Profile of Volleyball Player and its Possible Use for Injury Prevention', Sports Nutr Ther, vol. 02, no. 03, 2017, doi: 10.4172/2473-6449.1000128.

## Claims

1. A gel composition for topical application, comprising:
(i) a peptide component, consisting of a single-chain recombinant human peptide having a maximum length of 160 amino acids and comprising the amino acid sequence SEQ ID NO:1;
- high molecular weight (HMW) hyaluronic acid;
- panthenol;
- a gelling agent; and
- water.

2. The gel composition for topical application according to claim 1, wherein the aforementioned recombinant human peptide consists of the amino acid sequence SEQ ID NO:1.

3. The gel composition for topical application according to claim 1 or 2, wherein the high molecular weight (HMW) hyaluronic acid has a molecular weight greater than 1000 kDaltons, preferably between 1000 and 5000 kDaltons, and is optionally in salt form.

4. The gel composition for topical application according to claim 3, wherein the high molecular weight hyaluronic acid is in the salt form and is sodium hyaluronate.

5. The gel composition for topical application according to any one of claims 1 to 4, wherein the gelling agent is a polyacrylic acid polymer.

6. The gel composition for topical application according to any one of claims 1 to 5, also comprising further ingredients selected from the group consisting of thickening agents, wetting agents, solubilizing agents, chelating agents, pH adjusting agents, preservative agents, and any combination thereof.

7. The gel composition for topical application according to claim 6, comprising xanthan gum as the thickening agent.

8. The gel composition for topical application according to claim 6 or 7, comprising glycerin and propylene glycol or propanediol as the wetting agent(s).

9. The gel composition for topical application according to any one of claims 6 to 8, comprising PEG-40 hydrogenated castor oil as the solubilizing agent.

10. The gel composition for topical application according to any one of claims 6 to 9, comprising dehydroacetic acid, benzoic acid, ethylhexylglycerin and phenoxyethanol as the preservative agents.

11. The gel composition for topical application according to any one of claims 6 to 10, comprising EDTA disodium salt as the chelating agent.

12. The gel composition for topical application according to any one of claims 6 to 11, comprising sodium hydroxide as the pH adjusting agent.

13. The gel composition for topical application according to any one of claims 1 to 12, having a pH between 5.00 and 5.70.

14. The gel composition for topical application according to any one of claims 1 to 13, for use in the treatment of local inflammation.


SEQUENCE LISTING

<110> Contrad Swiss SA

<120> Composizione in gel per applicazione topica per la riduzione
dellinfiammazione locale

<130> E0131823-E6605-EC

<160> 1

<170> BiSSAP 1.3.6

<210> 1
<211> 160
<212> PRT
<213> Homo sapiens

<220>
<223> SH-Polipeptide 6

<400> 1
Ser Pro Gly Gln Gly Thr Gln Ser Glu Asn Ser Cys Thr His Phe Pro
1               5                   10                  15
Gly Asn Leu Pro Asn Met Leu Arg Asp Leu Arg Asp Ala Phe Ser Arg
            20                  25                  30
Val Lys Thr Phe Phe Gln Met Lys Asp Gln Leu Asp Asn Leu Leu Leu
        35                  40                  45
Lys Glu Ser Leu Leu Glu Asp Phe Lys Gly Tyr Leu Gly Cys Gln Ala
    50                  55                  60
Leu Ser Glu Met Ile Gln Phe Tyr Leu Glu Glu Val Met Pro Gln Ala
65                  70                  75                  80
Glu Asn Gln Asp Pro Asp Ile Lys Ala His Val Asn Ser Leu Gly Glu
                85                  90                  95
Asn Leu Lys Thr Leu Arg Leu Arg Leu Arg Arg Cys His Arg Phe Leu
            100                 105                 110
Pro Cys Glu Asn Lys Ser Lys Ala Val Glu Gln Val Lys Asn Ala Phe
        115                 120                 125
Asn Lys Leu Gln Glu Lys Gly Ile Tyr Lys Ala Met Ser Glu Phe Asp
    130                 135                 140
Ile Phe Ile Asn Tyr Ile Glu Ala Tyr Met Thr Met Lys Ile Arg Asn
145                 150                 155                 160